# EUROPÄISCHE PATENTSCHRIFT

(11) **EP 0 718 035 B1**
(45) Veröffentlichungstag und Bekanntmachung des Hinweises auf die Patenterteilung: **24.10.2001**
(21) Anmeldenummer: 95120019.5
(22) Anmeldetag: 19.12.1995
(51) Int. Cl.: B01J 13/00

(54) **Verfahren und Vorrichtung zur Herstellung wirkstoffhaltiger Dispersionen**
Method and apparatus for preparing dispersions containing active substances
Procédé et dispositif de fabrication de dispersions contenant des substances actives

(30) Priorität: 19.12.1994 DE 4445341
(43) Veröffentlichungstag der Anmeldung: 26.06.1996
(60) Teilanmeldung: 00114070.6
(73) Patentinhaber: BASF AKTIENGESELLSCHAFT, 67056 Ludwigshafen (DE)
(72) Erfinder: Riede, Thomas, Dr., D-64625 Bensheim (DE); Göbel, Werner, D-67149 Meckenheim (DE); Lockemann, Christian, Dr., D-68161 Mannheim (DE)
(74) Vertreter: Isenbruck, Günter, Dr.

(56) Entgegenhaltungen:
- WO-A-93/17665

## Beschreibung

Die Erfindung bezieht sich auf ein Verfahren und eine Vorrichtung zur Herstellung wirkstoffhaltiger Dispersionen.

In vielen Bereichen der chemischen Industrie müssen heute wirkstoffhaltige Dispersionen hergestellt werden, z. B. in der Farben- und in der Pharmaindustrie. Dabei ist es oft nicht möglich, den Wirkstoff, z. B. den Farbstoff oder den Pharmawirkstoff, unmittelbar in einer wäßrigen Flüssigkeit zu dispergieren. Insbesondere liegen die Wirkstoffpartikel dann nicht in der gewünschten mikronisierten Form vor.

Die Herstellung von feinstverteilten, mikronisierten Carotinoiden in einer wäßrigen Dispersion wird von der deutschen Offenlegungssschrift 29 43 267 beschrieben. Dort wird ein in einem Autoklaven befindliches Carotinoid in einem überkritischen Gas gelöst und die erhaltene Lösung in einer geeigneten wäßrigen kolloidalen Matrix, die sich in einem zweiten Autoklaven befindet, mittels eines Rührorgans dispergiert. Die so erhaltene Dispersion besteht aus wirkstoffhaltigen Tröpfchen von fluidem Gas in Wasser. Dieses zweiphasige System wird entspannt, wobei das Gas kontinuierlich aus dem Autoklaven abgezogen wird, die Flüssigkeit hingegen nur diskontinuierlich nach Beendigung des Prozesses entnommen werden kann. Durch dieses Verfahren wird eine mittlere Teilchengröße des Carotinoids von weniger als 1 Mikron erreicht. Nachteilig ist, daß es während des Entspannungsvorgangs zur Entmischung der Dispersion kommen kann, so daß der Wirkstoff nicht in ausreichender Weise von der flüssigen Matrix stabilisiert wird. Außerdem ist ein Wirkstoffverlust hinzunehmen.

Das europäische Patent EP 0 322 687 schlägt ein Verfahren zur Herstellung einer Arzneiform vor, die einen Wirkstoff und einen Träger umfaßt. Dabei werden ein fluides Gas, ein Wirkstoff und Trägermaterialien, die auch in einer Flüssigkeit gelöst sein können, in einen Sprühturm eingebracht, so daß das fluide Gas Wirkstoff und Träger aufnimmt. In einem Abscheider wird anschließend das fluide Gas von der entstandenen Wirkstoff/Träger-Kombination getrennt, so daß diese aus dem Separator entnommen werden kann. Bei diesem Verfahren werden trockene Wirkstoffpartikel erzeugt, nicht jedoch flüssige Dispersionen. Unter "fluidem Gas" wird eine bei Atmosphärendruck als Gas oder Dampf vorliegende Substanz verstanden, die bis in die Nähe ihres kritischen Punkts oder darüber hinaus verdichtet ist und daher als unter- oder überkritisches Fluid vorliegt.

Aufgabe der Erfindung ist die Bereitstellung eines Verfahrens und einer Vorrichtung zur Herstellung wirkstoffhaltiger Dispersionen, die zu einer besonders feinen Verteilung des Wirkstoffes in der Dispersion führen. Insbesondere sollen die mikronisierten Wirkstoffe nicht agglomerieren.

Diese Aufgabe wird durch das in den Ansprüchen beschriebene Verfahren gelöst. Dabei wird ein Wirkstoff in einem fluiden Gas gelöst und das wirkstoffbeladene fluide Gas in einer Flüssigkeit feinverteilt und dabei im wesentlichen vollständig gelöst. Während des Lösevorgangs des fluiden Gases wird der Feststoff gebildet. In den einzelnen, feinverteilten Gasblasen ist jeweils nur wenig Feststoff enthalten, so daß die dort entstehenden Feststoffpartikeln sehr klein sind. Da die Partikel unmittelbar in die Flüssigkeit übergehen, wo sie vorzugsweise in Schutzkolloide eingebunden werden, und zuvor in den einzelnen, feinverteilten Gasblasen jeweils nur wenig Feststoff enthalten ist, ist eine Agglomeration nahezu auszuschließen. Danach wird die Lösung entspannt und so wird das Gas von der wirkstoffbeladenen Flüssigkeit getrennt. Vorzugsweise wird ein Teil der abgetrennten wirkstoffbeladenen Flüssigkeit als Lösungsflüssigkeit für das fluide Gas wiederverwendet. Dadurch kann eine hohe Konzentration der Mikropartikel in der Dispersion unabhängig von der Wirkstofflöslichkeit im fluiden Gas erreicht werden. Weiter bevorzugt ist ein Verfahren, bei dem die Lösung bei der Entspannung nicht vollständig auf Atmosphärendruck entspannt wird, so daß die wirkstoffbeladene wiederverwendete Flüssigkeit mit dem fluiden Gas vorbeladen ist. Dadurch kann sowohl die Bildungsgeschwindigkeit der Feststoffpartikel als auch deren Morphologie beeinflußt werden. Durch die vollständige Auflösung des Gases in der Flüssigkeit ist darüber hinaus gewährleistet, daß der Wirkstoff vollständig aus dem Gas in die Flüssigkeit übergeht. Das Verfahren kann kontinuierlich oder diskontinuierlich durchgeführt werden, wobei in beiden Fällen eine Agglomeration von Teilchen, vorzugsweise aufgrund der Stabilisierung durch Schutzkolloide, nicht oder nur in vernachlässigbarem Ausmaß auftritt.

Bevorzugt ist die Rückführung des abgetrennten Gases, das als fluides Gas zur Lösung des Wirkstoffes wiederverwendet wird. Vorzugsweise besteht der verwendete Wirkstoff aus einer Mischung aus mehreren Wirkstoffen. Bevorzugte Wirkstoffe sind Farbstoffe, Vitamine, Carotinoide, Polymere, Liposome, Pharmawirkstoffe oder Pflanzenschutzwirkstoffe. Bevorzugte fluide Gase sind CO₂, N₂O, Ethylen, Propan, H₂O, NH₃, Kohlenwasserstoffe, Alkohole und Mischungen aus diesen Substanzen. Vorzugsweise wird als Flüssigkeit Wasser oder ein organisches Lösungsmittel verwendet, das vorzugsweise mit Zusätzen von Schutzkolloiden wie Tensiden, Polymeren, Cellulosen, Dextrinen oder Proteinen wie Gelatine und/oder Emulgatoren oder ähnlichem angereichert ist.

Die Aufgabe der Erfindung wird auch durch die in den Ansprüchen beschriebene Vorrichtung gelöst. Eine Vorrichtung nach den Vorrichtungsansprüchen beinhaltet einen Extraktor für die Auflösung eines Wirkstoffes in einem fluiden Gas, der über eine Leitung mit einem Mischorgan für die Auflösung des fluiden wirkstoffbeladenen Gases in einer Flüssigkeit verbunden ist. Die Flüssigkeit wird dem Mischorgan über eine weitere Leitung zugeführt. Die Vorrichtung enthält darüber hinaus eine Verweilzeitstrecke, die mit einem Abscheider verbunden ist, in dem das Gas von der wirkstoffhaltigen Flüssigkeit getrennt wird. Der Abscheider weist Ableitungen für das abgetrennte Gas und für die abgetrennte Flüssigkeit auf. Außerdem enthält die Vorrichtung eine Rückführung für einen Teil der abgetrennten Flüssigkeit zum Mischorgan.

Zwischen der Verweilzeitstrecke und dem Abscheider ist ein Entspannungsventil angebracht. In einer bevorzugten Ausführungsform führt die Ableitung für das abgetrennte Gas dieses aus dem Abscheider zum Extraktor zurück.

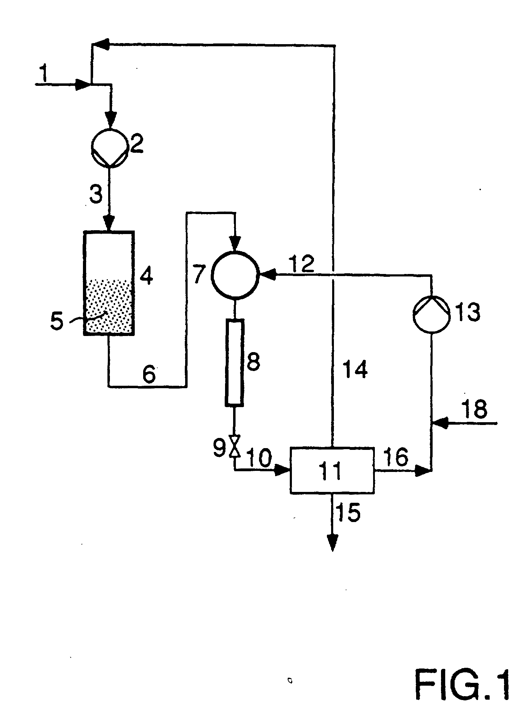

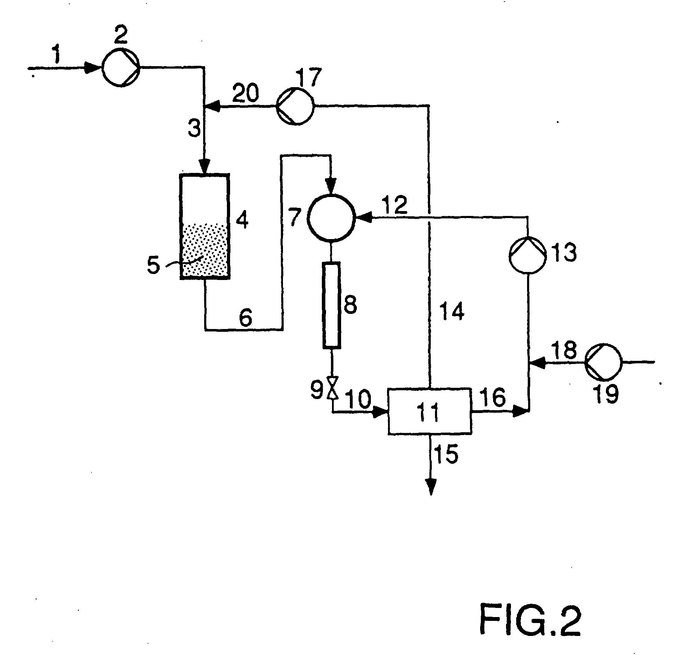

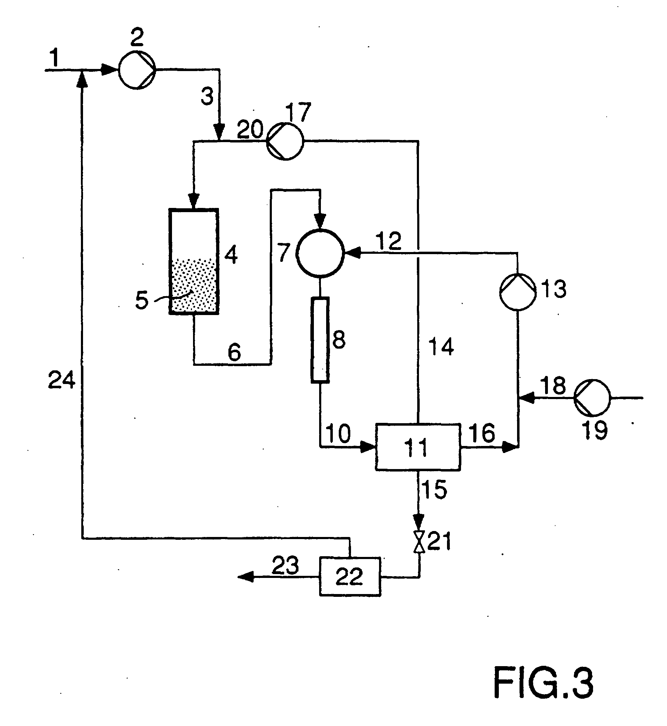

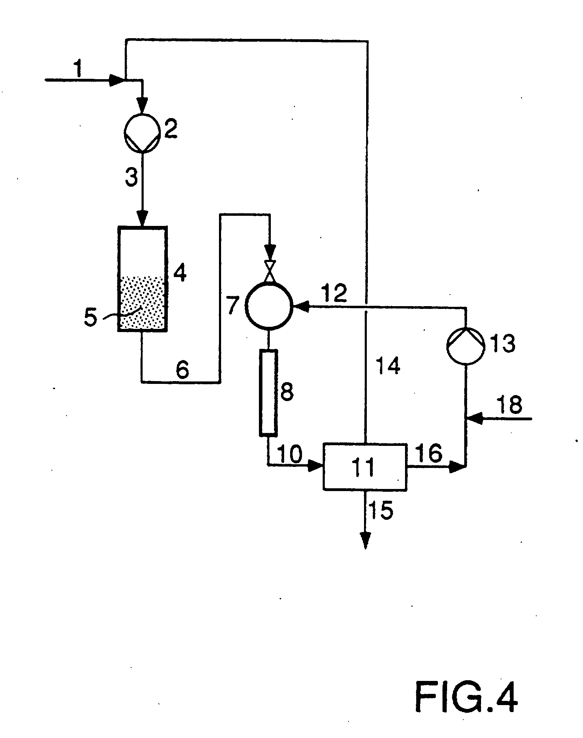

Die Erfindung wird im folgenden anhand der Figuren 1 und 2 beschrieben. Es zeigen:
- Fig. 1:: Versuchsaufbau für eine erste Verfahrensvariante,
- Fig. 2:: Versuchsaufbau für eine zweite Verfahrensvariante,

In Fig. 1 wird ein Gas 1 mit Hilfe eines Verdichters 2 in den überkritischen, d. h. fluiden Zustand gebracht. Dieses fluide Gas 3 nimmt in einem Extraktor 4 den Wirkstoff 5 auf. Das wirkstoffbeladene fluide Gas wird sodann über Leitung 6 zum Mischorgan 7 geführt, wo es mit einer Flüssigkeit vermischt wird, die über Leitung 12 dem Mischorgan zugeführt wird. Im Mischorgan 7 und in der anschließenden Verweilzeitstrekke 8 wird das wirkstoffbeladene fluide Gas in der Flüssigkeit im wesentlichen vollständig aufgelöst. Der in dem fluiden Gas gelöste Wirkstoff fällt in Form feinstverteilter Teilchen mit Größen im Bereich kleiner 1 *µ*m in die Flüssigkeit aus. Die so entstandene Wirkstoff/Flüssigkeitsdispersion wird durch das Ventil 9 entspannt und über Leitung 10 dem Abscheider 11 zugeführt. Ein Teil der wirkstoffbeladenen Flüssigkeit wird über Leitung 15 abgeführt, der andere Teil wird über Leitung 16 und Verdichter 13 zusammen mit frischer Flüssigkeit, die mit Leitung 18 zugeführt wird, über Leitung 12 zum Mischorgan 7 geleitet. Durch diese partielle Rückführung kann die Konzentration der Wirkstoffpartikel in der Dispersion unabhängig von der Wirkstofflöslichkeit in dem fluiden Gas bestimmt werden. Das abgetrennte Gas wird über Leitung 14 abgeführt und dem Kreislauf vor dem Verdichter 2 wieder zugeführt. Die Lösung kann am Ende der Verweilzeitstrecke vollständig entspannt oder aber auf einen Druck oberhalb des Atmosphärendrucks entspannt werden.

Durch eine unvollständige Entspannung, wie in Fig. 2 dargestellt, bleibt die Flüssigkeit mit fluidem Gas vorbeladen, wodurch die Kristallisationsgeschwindigkeit bei der Bildung der Wirkstoffpartikeln ebenso wie deren Morphologie beeinflußt werden kann. Die Rückführung des Gases erfolgt in diesem Fall dadurch, daß das abgetrennte Gas über Leitung 14 abgeführt, im Verdichter 17 auf Systemdruck verdichtet und dem Kreislauf über Leitung 20 nach dem Verdichter 2 wieder zugeführt wird. Frische Flüssigkeit wird im Verdichter 19 verdichtet und dem Kreislauf über Leitung 18 zugeführt.

### Beispiel 1

In einem temperierbaren Autoklav mit 250 ml Inhalt wurden etwa 5 g β-Carotin vorgelegt. Ein N₂O-Strom (250 bar - 0,8 Kg pro Stunde) wurde auf 45°C vorgewärmt und in den Autoklav geleitet. Der mit Carotin beladene N₂O-Strom wurde dann über eine thermostatisierte Leitung zu einer Mischdüse geführt. Dort wurde der Gasstrom in einer wäßrigen Lösung, die 3,8 Gew.-% Gelatine enthielt, dispergiert. Dabei betrug die Temperatur 45°C, der Druck 250 bar. Anschließend durchlief die Dispersion eine Verweilzeitstrecke, wobei sich das Gas vollständig in der wäßrigen Lösung löste. In einem Abscheider wurde schließlich die Dispersion entspannt und das desorbierende Gas von der Lösung getrennt. Die Flüssigkeit wurde anschließend wieder auf 250 bar verdichtet und zur Mischdüse zurückgeführt. Die Teilchengröße des in dem Endprodukt enthaltenden Carotins lag deutlich unter 1 *µ*m.

## Patentansprüche

1. Verfahren zur Herstellung wirkstoffhaltiger flüssiger Dispersionen, bei dem man einen Wirkstoff in einem fluiden Gas auflöst, **dadurch gekennzeichnet, daß** man die erhaltene Lösung in einer Flüssigkeit, die ein Nichtlöser für den Wirkstoff ist, fein verteilt, wobei das fluide Gas im Wesentlichen vollständig in der Flüssigkeit aufgelöst und der Wirkstoff in feinteiliger Form ausgeschieden wird, die erhaltene Dispersion dann entspannt und das gebildete Gas von der mit festem Wirkstoff beladenen Flüssigkeit trennt.

2. Verfahren nach Anspruch 1, **dadurch gekennzeichnet, daß** ein Teil der abgetrennten mit Wirkstoff beladenen Flüssigkeit als Lösungsflüssigkeit für das fluide Gas verwendet wird.

3. Verfahren nach Anspruch 2, **dadurch gekennzeichnet, daß** die Lösung bei der Entspannung nicht vollständig auf Atmosphärendruck entspannt wird, so daß die mit Wirkstoff beladene wiederverwendete Flüssigkeit mit dem fluiden Gas vorbeladen ist.

4. Verfahren nach einem der vorhergehenden Ansprüche, **dadurch gekennzeichnet, daß** das abgetrennte Gas zurückgeführt und als fluides Gas zur Lösung des Wirkstoffs wiederverwendet wird.

5. Verfahren nach einem der vorhergehenden Ansprüche, **dadurch gekennzeichnet, daß** der Wirkstoff aus einer Mischung aus mehreren Wirkstoffen besteht.

6. Verfahren nach einem der vorhergehenden Ansprüche, **dadurch gekennzeichnet, daß** als Wirkstoff ein Farbstoff, ein Vitamin, ein Carotinoid, ein Polymeres, ein Liposom, ein Pharmawirkstoff oder ein Pflanzenschutzwirkstoff, insbesondere ein Protein oder Peptid eingesetzt wird.

7. Verfahren nach einem der vorhergehenden Ansprüche, **dadurch gekennzeichnet, daß** als fluides Gas CO₂, N₂O, Ethylen, Propan, H₂O, NH₃, ein Kohlenwasserstoff, ein Alkohol oder eine Mischung aus diesen Substanzen eingesetzt wird.

8. Verfahren nach einem der vorhergehenden Ansprüche, **dadurch gekennzeichnet, daß** als Flüssigkeit Wasser oder ein organisches Lösungsmittel, vorzugsweise mit Zusätzen von Tensiden, Polymeren, Cellulosen, Dextrinen oder Proteinen wie Gelatine und/oder Emulgatoren eingesetzt wird.

9. Vorrichtung zur Durchführung des Verfahrens nach einem der Ansprüche 1 bis 8, **gekennzeichnet durch**
einen Extraktor (4) für die Auflösung eines Wirkstoffes (5) in einem fluiden Gas,
ein mit dem Extraktor (4) über eine Leitung (6) verbundenes Mischorgan (7) für die Auflösung des fluiden wirkstoffbeladenen Gases in einer Flüssigkeit, die über eine Leitung (12) dem Mischorgan zugeführt wird,
eine Verweilzeitstrecke (8), die über ein Entspannungsventil (9) mit einem Abscheider (11) verbunden ist, in dem das Gas von der wirkstoffhaltigen Flüssigkeit getrennt wird,
eine Ableitung (14) für das abgetrennte Gas, sowie eine Ableitung (15) für die abgetrennte Flüssigkeit aus dem Abscheider, und
eine Rückführung (16) für einen Teil der abgetrennten Flüssigkeit zum Mischorgan (7).

10. Vorrichtung nach Anspruch 9, **dadurch gekennzeichnet, daß** die Leitung (14) das abgetrennte Gas aus dem Abscheider (11) zum Extraktor (4) zurückführt.

## Claims

1. A process for preparing active ingredient-containing liquid dispersions, in which an active ingredient is dissolved in a fluid gas, wherein the resulting solution is finely dispersed in a liquid which is a nonsolvent for the active ingredient, the fluid gas being essentially completely dissolved in the liquid, and the active ingredient being deposited in fine-particle form, the resulting dispersion is then decompressed and the gas which forms is separated from the liquid loaded with solid active ingredient.

2. A process as claimed in claim 1, wherein part of the liquid loaded with active ingredient which has been separated off is used as liquid for dissolving the fluid gas.

3. A process as claimed in claim 2, wherein the solution is not completely decompressed to atmospheric pressure on decompression, so that the reused liquid loaded with active ingredient is preloaded with the fluid gas.

4. A process as claimed in any of the preceding claims, wherein the gas which has been separated off is recycled and reused as fluid gas to dissolve the active ingredient.

5. A process as claimed in any of the preceding claims, wherein the active ingredient consists of a mixture of a plurality of active ingredients.

6. A process as claimed in any of the preceding claims, wherein a dye, a vitamin, a carotenoid, a polymer, a liposome, an active pharmaceutical ingredient or a crop protection agent, in particular a protein or peptide, is used as active ingredient.

7. A process as claimed in any of the preceding claims, wherein CO₂, N₂O, ethylene, propane, H₂O, NH₃, a hydrocarbon, an alcohol or a mixture of these substances is used as fluid gas.

8. A process as claimed in any of the preceding claims, wherein water or an organic solvent, preferably with additions of surfactants, polymers, celluloses, dextrins or proteins such as gelatin and/or emulsifiers is used as liquid.

9. An apparatus for carrying out the process as claimed in any of claims 1 to 8, which comprises an extractor (4) to dissolve an active ingredient (5) in a fluid gas, a mixer (7) which is connected via a line (6) to the extractor (4) to dissolve the fluid gas loaded with active ingredient in a liquid, which is fed to the mixer via a line (12), a holdup section (8) which is connected via a decompression valve (9) to a separator (11) in which the gas is separated from the liquid containing active ingredient, a discharge line (14) for the gas which has been separated off, and a discharge line (15) for the liquid which has been separated off from the separator, and a return line (16) for part of the liquid which has been separated off to the mixer (7).

10. An apparatus as claimed in claim 9, wherein the line (14) returns the gas which has been separated off from the separator (11) to the extractor (4).

## Revendications

1. Procédé de préparation de dispersions liquides contenant des matières actives, dans lequel on dissout une matière active dans un gaz fluide, **caractérisé en ce qu'**on répartit finement la solution obtenue dans un liquide qui est un non-solvant pour la matière active, le gaz fluide se dissolvant pour ainsi dire complètement dans le liquide, et la matière active étant séparée sous forme finement divisée, puis on détend la dispersion ainsi obtenue, et le gaz formé est séparé du liquide chargé de la matière active solide.

2. Procédé selon la revendication 1, **caractérisé en ce qu'**une partie du liquide séparé, chargé de matière active, est utilisée en tant que liquide solvant pour le gaz fluide.

3. Procédé selon la revendication 2, **caractérisé en ce que** la solution, lors de la détente, n'est pas complètement détendue à la pression atmosphérique, de sorte que le liquide réutilisé, chargé de matière active, est pré-chargé du gaz fluide.

4. Procédé selon l'une des revendications précédentes, **caractérisé en ce que** le gaz séparé est renvoyé en recyclage et est réutilisé en tant que gaz fluide pour dissoudre la matière active.

5. Procédé selon l'une des revendications précédentes, **caractérisé en ce que** la matière active est constituée d'un mélange de plusieurs matières actives.

6. Procédé selon l'une des revendications précédentes, **caractérisé en ce qu'**on utilise en tant que matière active un colorant, une vitamine, un caroténoïde, un polymère, un liposome, un principe actif pharmaceutique ou une matière active phytosanitaire, en particulier une protéine ou un peptide.

7. Procédé selon l'une des revendications précédentes, **caractérisé en ce qu'**on utilise en tant que gaz fluide le CO₂, le N₂O, l'éthylène, le propane, le H₂O, le NH₃, un hydrocarbure, un alcool ou un mélange de ces substances.

8. Procédé selon l'une des revendications précédentes, **caractérisé en ce qu'**on utilise en tant que liquide l'eau ou un solvant organique, de préférence additionné de tensioactifs, de polymères, de celluloses, de dextrines ou de protéines telles que la gélatine et/ou d'émulsifiants.

9. Appareillage pour mettre en oeuvre le procédé selon l'une des revendications 1 à 8, **caractérisé par**
un extracteur (4) pour dissoudre une matière active (5) dans un gaz fluide,
un organe mélangeur (7), relié à l'extracteur (4) par une conduite (6), pour dissoudre le gaz fluide chargé de matière active dans un liquide qui par une conduite (12) est envoyé à l'organe mélangeur,
une zone à temps de séjour (8), qui par l'intermédiaire d'une soupape de détente (9) est reliée à un séparateur (11) dans lequel le gaz est séparé du liquide contenant la matière active,
une conduite d'évacuation (14) pour le gaz séparé, ainsi qu'une conduite d'évacuation (15) pour le liquide séparé sortant du séparateur, et
une conduite (16) pour renvoyer en recyclage une partie du liquide séparé vers l'organe mélangeur (7).

10. Appareillage selon la revendication 9, **caractérisé en ce que** la conduite (14) renvoie à l'extracteur (4) le gaz séparé sortant du séparateur (11).
